# EUROPÄISCHE PATENTANMELDUNG

(11) **EP 0 142 629 A1**
(43) Veröffentlichungstag der Anmeldung: **29.05.1985**
(21) Anmeldenummer: 84109650.6
(22) Anmeldetag: 14.08.1984
(51) Int. Cl.: C07D 403/12, C07D 413/12, A01N 47/36

(54) **Neue Pyrazolyl- und Isoxazolylsulfonylharnstoffe**

(30) Priorität: 25.08.1983 DE 3330602
(71) Anmelder: HOECHST AKTIENGESELLSCHAFT, 65926 Frankfurt am Main (DE)
(72) Erfinder: Willms, Lothar, Dr., D-5463 Unkel (DE); Boesenberg, Heinz, Dr., D-6238 Hofheim am Taunus (DE); Bauer, Klaus, Dr., D-6054 Rodgau (DE); Bieringer, Hermann, Dr., D-6239 Eppstein/Taunus (DE); Bürstell, Helmut, Dr., D-6000 Frankfurt am Main 50 (DE)

(57) **Zusammenfassung**

Verbindungen der Formel (I), 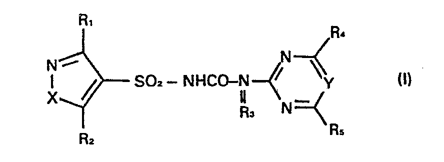 worin
X Sauerstoff oder NR₆, Y CH oder Stickstoff, R., R₂ Wasserstoff, Halogen (subst.) Alkyl, (subst.) Alkenyl, (subst.) Alkinyl, OH, (subst.) Alk(en) (in)yloxy, Reste von AlkanCarbonsäuren, -Sulfonsäuren, -Sulfen- und -Sulfinsäuren und von deren Amiden, (subst.) Phenyl, Benzyl, (subst.) Hydroxycarbonyl, (subst.) Aminocarbonyl, R₃ Wasserstoff oder Alkyl, R₄, R₅ Wasserstoff, (subst.) Alkyl oder (subst.) Alkoxy, Halogen, Alkylthio, Mono- oder Dialkylamino sowie deren Salze besitzen vorteilhafte herbizide Wirkung gegenüber mono- oder dikotylen Schadpflanzen und eignen sich daher zum Einsatz in Großkulturen. Sie besitzen außerdem pflanzenwachstumsregulierende Wirkung.

## Beschreibung

Heterocyclisch substituierte Phenylsulfonylharnstoffe, die herbizide oder pflanzenwuchsregulierende Eigenschaften aufweisen, sind beispielsweise aus NL-PS 121 788, DE-OS 27 15 786, EP-PS 1 485 bekannt.

Diese weisen jedoch Nachteile bei der Anwendung auf wie beispielsweise eine hohe Persistenz oder unzureichende Selektivität.

Es wurden nun neue Pyrazol-4-yl- und Isoxazol-4-yl-sulfonylharnstoffe gefunden, die als weitere heterocyclische Komponente einen Pyrimidin- oder Triazinring enthalten. Diese besitzen vorteilhafte herbizide und pflanzenwachstumsregulierende Eigenschaften.

Gegenstand der vorliegenden Erfindung sind daher Verbindungen der allgemeinen Formel (I), 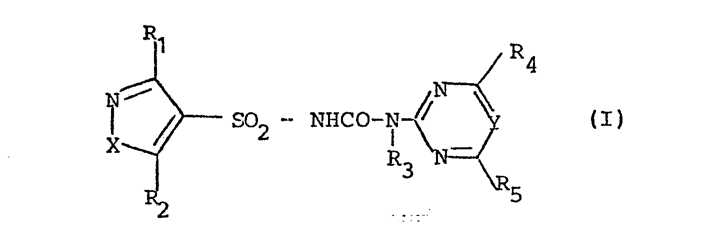 worin
X Sauerstoff oder NR₆,
Y CH oder Stickstoff,
R₁ und R₂ unabhängig voneinander Wasserstoff, Halogen, . (C₁-C₆)Alkyl, (C₂-C₆)Alkenyl, (C₂-C₆)Alkinyl, die gegebenenfalls durch ein bis drei Halogenatome, (C₁-C₄)Alkoxy, (C₁-C₄)Alkylsulfenyl, (C₁-C₄) Alkylsulfinyl, (C₁-C₄) Alkylsulfonyl oder (C₁-C₄) Alkoxycarbonyl substituiert sind, OH, gegebenenfalls halogeniertes (C₁-C₄)Alkoxy, (C₂-C₄) Alkenyloxy, (C₂-C₄) Alkinyloxy , -OSO₂ (C₁-C₄)Alkyl, -OCO(C₁-C₄)Alkyl, -OCON [(C₁-C₄)Alkyl]₂, -OCONH(C₁-C₄)Alkyl, -OSO₂-C₆H₄-(4)-CH₃, -OSO₂N(CH₃)₂, -OCO-C₆H₅, (C₁-C₄)Alkylsulfenyl, (C₁-C₄)Alkylsulfinyl, (C₁-C₄)Alkylsulfonyl, -SO₂NH((C₁-C₄)Alkyl, -SO₂N[(C₁-C₄)Alkyl]₂, einen Phenylrest, der gegebenenfalls durch Halogen, CF₃, (C₁-C₄)Alkyl, (C₁-C₄)Alkoxy, (C₁-C₄) Alkoxycarbonyl, (C₁-C₄)-Alkylsulfenyl, (C₁-C₄) Alkylsulfinyl oder (C₁-C₄)-Alkylsulfonyl substituiert ist, Benzyl, (C₁-C₄)Alkoxycarbonyl, (C₂-C₄) Alkenyloxycarbonyl, (C₂-C₄)Alkinyloxycarbonyl, Aminocarbonyl, (C₁-C₄)Alkylaminocarbonyl oder Di (C₁-C₄-alkyl)aminocarbonyl, R₃ Wasserstoff oder (C₁-C₄)Alkyl,
R_{4'} R₅ unabhängig voneinander Wasserstoff, einen(C₁-C₄) Alkyl oder (C₁-C₄)Alkoxyrest, die gegebenenfalls ein bis dreifach durch Halogen oder 1 bis zweifach durch (C₁-C₂)Alkoxy oder (C₁-C₂)Alkylthio substituiert sein können, Halogen, (C₁-C₄)Alkylthio, (C₁-C₄) Alkylamino oder (C₁-C₄)Dialkylamino,

und für X = NR₆
R₆ Wasserstoff, (C₁-C₆)Alkyl, (C₂-C₄)Alkenyl, (C₂-C₄)Alkinyl, einen Phenylrest, der gegebenenfalls durch Halogen, (C₁-C₄)Alkyl, CF₃, (C₁-C₄) Alkoxycarbonyl oder N0₂ substituiert ist, -CO(C₁-C₄) Alkyl, -COO(C₁-C₆)-Alkyl, -CONH(C₁-C₆)Alkyl, -CON[(C₁-C₄)Alkyl]₂, -CO-C₆H₅, -CH₂-COO(C₁-C₄) Alkyl, -SO₂(C₁-C₄)Alkyl oder -SO₂-C₆H₄-(4)CH₃ bedeuten sowie deren nichtphytotoxische Salze.
   Die neuen Verbindun.gen der allgemeinen Formel I lassen sich aus an sich bekannten oder nach bekannten Verfahren hergestellten Ausgangsmaterialien synthetisieren.
   Die Herstellungsverfahren sind dadurch gekennzeichnet, daß man
   a) Verbindungen der Formel (II) 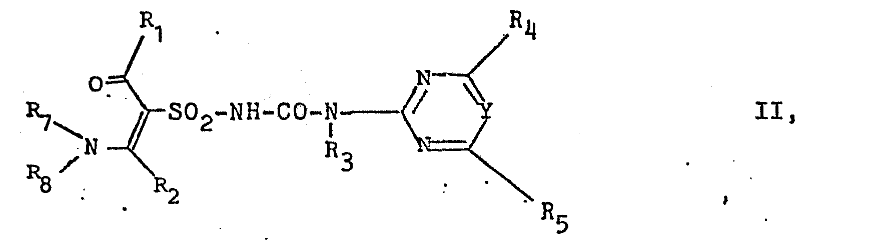 worin
R₇ und R₈ unabhängig voneinander Wasserstoff, (C₁-C₈)Alkyl (C₃-C₇)-Cycloalkyl, Phenyl, welches gegebenenfalls ein- oder mehrfach durch Halogen, (C₁-C₄) Alkyl, (C₁-C₄)-Alkoxy, (C₁-C₄)Alkylthio, (C₁-C₄)Alkoxycarbonyl, CF₃, NO₂ oder CH₃SO₂-substituiert sein kann, oder die Reste R₇ und R₈ zusammen mit dem gemeinsamen Stickstoffatom einen 5- bis 7-gliedrigen aliphatischen Ring bilden, worin eine CH₂-Gruppe gegebenenfalls durch Sauerstoff, NH oder N(C₁-C₄)Alkyl ersetzt sein kann,
R₉ Wasserstoff, (C₁-C₈)Alkyl, (C₃-C₇)-Cycloalkyl oder Phenyl, welches gegebenenfalls ein-oder mehrfach durch Halogen, (C₁-C₄)Alkyl, (C₁-C₄)Alkoxy, (C₁-C₄)Alkylthio, CF₃, N0₂ oder (C₁-C₄)Alkoxycarbonyl substituiert sein kann und
R₂, R₃, R₄, R₅ und X die Bedeutungen wie in Formel (I) besitzen,

mit Verbindungen der Formel (III) 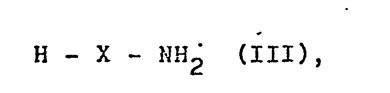 worin X die Bedeutung wie in Formel I besitzt, umsetzt oder oderb) gegebenenfalls Verbindungen der Formel I mit X= NH oder R₂= OH durch Derivatisierung in andere Verbindungen der Formel I überführt. Verbindungen der Formel I mit X= NH können beispielsweise in bekannter Weise durch Alkylierung oder Acylierung der NH-Gruppe des Pyrazolringes in andere Verbindungen der Formel I überführt werden,während Verbindungen der Formel I mit R₂= OH nach üblichen Methoden durch Alkylierung, Acylierung oder Halogenierung der heterocyclischen OH-Gruppierung in andere Verbindungen der Formel (I) umgewandelt werden können.

Zu a) Die Umsetzung der Verbindungen (II) und (II) erfolgt vorzugsweise in inerten Lösungsmittel wie aliphatischen oder aromatischen Kohlenwasserstoffen, beispielsweise Xylol oder Toluol, Säurenitrilen wie Acetonitril, halogenierten oder nitrierten Aliphaten wie Dichlormethan, Chloroform, Nitromethan, Säureamiden wie Dimethylformamid, aliphatischen Alkoholen wie Methanol oder Ethanol, Heterocyclen wie Tetrahydrofuran oder in Gemischen dieser Lösungsmittel mit Wasser gegebenenfalls unter Zusatz von basischen Verbindungen wie Alkaliacetaten, -carbonaten beispielsweise -Natriumacetat, Kaliumcarbonat oder tertiären organischen Aminen wie Trieethylamin, Pyridin. Die Reaktion wird bei-Temperaturen zwischen 0°C und der Siedetemperatur des Lösungsmittels durchgeführt. Die Verbindungen der Formel (III) werden gegebenenfalls in zwei- bis dreifachem molarem Überschuß, bezogen auf (I), eingesetzt.
.Zu b) Die Alkylierung oder Acylierung wird vorzugsweise in inerten Lösungsmitteln wie Dimethylformamid, Acetonitril, Tetrahydrofuran, Kohlenwasserstoffen wie Xylol oder Wasser bei Temperaturen zwischen -20 C und der Siedetemperatur des Lösungsmittels durchgeführt. Gegebenenfalls wird unter Zusatz von basischen Verbindungen, wie Alkalihydroxiden, -carbonaten beispielsweise Natirumhydroxid, Kaliumcarbonat oder tertiären organischen Aminen wie Triethylamin gearbeitet. Als Alkylierungsmittel werden übliche Mittel wie Dimethylsulfat, Ethylbromid oder Propargylbromid, als Acylierungsmittel übliche Verbindungen wie Acetanhydrid, Chlorameisensäureetlnylester, Dimethylcarbamidsäurechlorid oder Methylisocyanat eingesetzt.

Die Verbindungen der Formel (I) können Salze bilden, bei denen das Sulfonamid-Wasserstoffatom durch ein geeignetes Kation ersetzt ist. Diese Salze sind im allgemeinen Metall-, gegebenenfalls substituierte Amnonium- oder anorganische Aminsalze. Diese werden vorzugsweise in inerten Lösungsmitteln wie z.B. Wasser, Methanol oder Aceton bei Tem^{p}era- turen von 20 - 100°C hergestellt. Geeignete Basen zur Herstellung der erfindungsgemäßen Salze sind z.B. Alkalicarbonate wie Kaliumcarbonat, Ammoniak oder tertiäre organische Amine wie Ethanolamin.

Die Ausgangsstoffe der Formel III sind bekannt oder lassen sich nach im Prinzip bekannten Verfahren herstellen.

Die als Ausgangsmaterialien verwendeten Verbindungen der Formel II sind Gegenstand der Deutschen Patentanmeldung P 33 30 603.6.

Die erfindungsgemäßen heterocyclischen Sulfonylharnstoffderivate weisen eine ausgezeichnete herbizide Wirkung und in wichtigen Großkulturen eine sehr gute Selektivität auf. Sie eignen sich daher zur selektiven Bekämpfung zweikeimblättriger und grasartiger annueller und perennierender Unkräuter, insbesondere in landwirtschaftlich bedeutenden Kulturen wie z.B. Weizen, Gerste, Roggen, Reis, Mais, Zuckerrübe und Soja. Dabei ist es gleichgültig, ob die Substanzen in Vorsaat-, Vorauflauf- oder Nachauflaufspritzung ausgebracht werden. Werden die erfindungsgemäβen Verbindungen vor dem Keimen der Unkrautpflanzen im Vorsaat- oder Vorauflaufverfahren auf die Erdoberfläche appliziert, so wird das Auflaufen der Keimlinge nicht verhindert. Die Unkräuter wachsen bis zum Keimblattstadium heran, stellen jedoch dann ihr Wachstum ein und sterben schließlich nach 3 - 5 Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen sch ädliche Unkrautkonkurrenz sehr früh und nachhaltig beseitigt wird.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltstoffen und zur Ernteerleichterung wie z.B. durch Auslösen von Desikkation und Wuchsstauchung eingesetzt werden. Des weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann. Besonders hervorzuheben ist die wachstumsregulatorische Wirksamkeit der Verbindungen als Wuchshemmer in Getreide, Mais, Soja, Baumwolle, Rasen sowie ihre Fähigkeiten, den Gehalt an erwünschten Inhaltsstoffen wie Kohlehydraten und Protein bei Nutzpflanzen zu erhöhen. Schließlich zeigen die Verbindungen eine sehr gute Verbesserung der Fruchtabszission, insbesondere bei Zitrusfrüchten, oder sie bewirken eine Reduktion der Haltekraft.

Gegenstand der Erfindung sind daher auch herbizide bzw. wachstumsregulierende Mittel, die gekennzeichnet sind durch einen Gehalt an einer Verbindung der Formel I in Kombination mit üblichen Formulierungshilfsmitteln und Inertstoffen sowie deren Verwendung im landwirtschaftlichen Bereich.

Die erfindungsgemäßen Mittel enthalten die Wirkstoffe der Formel I im allgemeinen zu 2 - 95 Gew.-%. Sie können als Spritzpulver, emulgierbare Konzentrate, versprühbare Lösungen, Stäubemittel oder Granulate in den üblichen Zubereitungen angewendet werden.

Die Spritzpulver sind in Wasser gleichmäßig dispergierbare Präparate, die neben dem Wirkstoff außer einem Verdünnungsmittel oder Inertstoff noch Netzmittel z.B. polyoxethylierte Alkylphenole, polyoxethylierte Fettalkohole, Alkyl- oder Alkylphenylsulfonate und Dispergiermittel, z.B. ligninsulfonsaures Natrium, 2,2'-dinaphthylmethan-6,6'-disulfon- saures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden:
Alkylarylsulfonsaure Kalziumsalze wie Ca-dodecyl-benzosulfonat oder nichtionische Emulgatoren wie Eettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyether, Sorbitanfettsäureester, Polyoxethylensorbitfett-säureester oder Polyoxethylen-sorbitester.

Stäubemittel erhält man durch Vermahlen des Wirkstoffes mit fein verteilten, festen Stoffen, z.B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranalien üblichen Weise - gewünschtenfalls in Mischung mit Düngemitteln - hergestellt werden.

Bei herbiziden Mitteln können die Konzentrationen der Wirkstoffe in den handelsüblichen Formulierungen verschieden sein.

In Spritzpulvern variiert die Wirkstoffkonzentration z.B. zwischen etwa 10 % und 80 %, der Rest besteht aus den oben angegebenen Formulierungszusätzen. Bei emulgierbaren Konzentraten kann die Wirkstoffkonzentration gleichfalls etwa 10 % bis 80 % betragen. Staubförmige' Formulierungen enthalten etwa 2 - 20 %. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Zur Anwendung als Herbizide werden die handelsüblichen Konzentrate gegebenenfalls in üblicher Weise verdünnt, z.B. bei Spritzpulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit, u.a. variiert die erforderliche Aufwandmenge. Sie beträgt im allgemeinen zwischen 0,01 und 10 kg/ha, vorzugsweise etwa 0,1 bis 5,0 kg/ha Wirkstoff.

Für einige Anwendungsgebiete kann es zweckmäßig sein, die neuen Herbizide mit einem oder mehreren Ilerbiziden gemeinsam anzuwenden, z.B. als Tankmischung oder in Form einer Fertigformulierung, um weitere vorteilhafte Wirkungen zu erzielen.

Die erfindungsgemäßen Wirkstoffe können mit anderen Herbiziden, Insektiziden und Fungiziden kombiniert werden.

Zur Anwendung als Wachstumsregulatoren eignen sich Konzentrationen zwischen 0,01 und 1,25 kg/ha. Bevorzugt verwendet man wäßrige Dispersionen von Spritzpulvern oder Verdünnungen von emulgierbaren Konzentraten. Die Anwendung erfolgt im Nachauflauf. Bevorzugte Kulturen sind Mais und Tabak.

### Herstellungsbeispiele

### Beispiel 1

### 1-(3,5-Dimethyl-isoxazol-4-yl-sulfonyl)-3-(4-methoxy-6-methyl-1,3,5-triazin-2-yl)-harnstoff

14,3g (0,04 mol) N-[(4-Methoxy-6-methyl-1, 3, 5-triazin-2-yl ) aminocarbonyl]-1-methylcarbonyl-2-methylamino-prop-1-en-1-yl-sulfonamid wurden in 150 ml Methanol bei 30°C suspendiert und nacheinander mit 7,2 g (0,088 mol) Natriumacetat und 6,16 g (0,088 mol) Hydroxylaminhydrochlorid versetzt. Man rührte 18 Std. bei Raumtemperatur nach, erwärmte 5 Min. auf 60°C, filtrierte, engte die Lösung ein und nahm in Wasser auf. Nach Ansäuern mit 2nHCl (pH 2-3) wurden 9,8 g (71,7 % d.Th.) des gewünschten Produkts erhalten. Fp. 173°C (Z.).

### Beispiel 2

### 1-(1,3,5-Trimethyl-pyrazol-4-yl-sulfonyl)-3-(4,6-dimethyl- pyrimidin-2-yl)-harnstoff

17,1 g (0,05 mol) N-[(4, 6-Dimethyl-pyrimidin-2-yl)-amino- carbonyl]-1-methylcarbonyl-2-methylamino-prop-1-en-1-yl- sulfonamid wurden in 150 ml Methanol supsendiert und bei 0°C mit 5,06 g (0,11 mol) Methylhydrazin versetzt. Man rührte 18 h bei Raumtemperatur nach und arbeitete wie unter Beispiel 1 auf. Man erhielt 14,3 g (85 % d.Th.) an 1-(1,3,5-Trimethyl-pyrazol-4-yl-sulfonyl)-3-(4,6-dimethyl-pyrimidin-2-yl)-harnstoff. Fp. 216-220°C (Z.).

### Beispiel 3

### 1-(1,3-Dimethyl-5-hydroxy-pyrazol-4-yl-sulfonyl)-3-(4,6-dimethylpyrimidin-2-yl)-harnstoff

18,55 g (0,05 mol) Ethyl-2-[ 3-(4,6-dimethyl-2-pyrimidinyl)-ureidosulfonyl,7-3-methylamino-2-butenoat wurden in 150 ml Methanol suspendiert und bei 0°C mit 5,06 g (0,11 mol) Methylhydrazin versetzt. Nach 18 h Rühren bei Raumtemperatur wurde wie unter Beispiel 1 aufgearbeitet. Man erhielt 14,7 g (86,4 % d.Th.) an 1-(1,3-Dimethyl-5-hydroxy-pyrazol-4-yl-sulfonyl)-3-(4,6-dimethyl-pyrimidin-2-yl)-harnstoff. Fp. 175-179⁰C (Z.).

Wie in Beispielen 1-3 beschrieben, lassen sich die in den nachfolgenden Tabellen 1 und 2 aufgeführten Verbindungen herstellen.

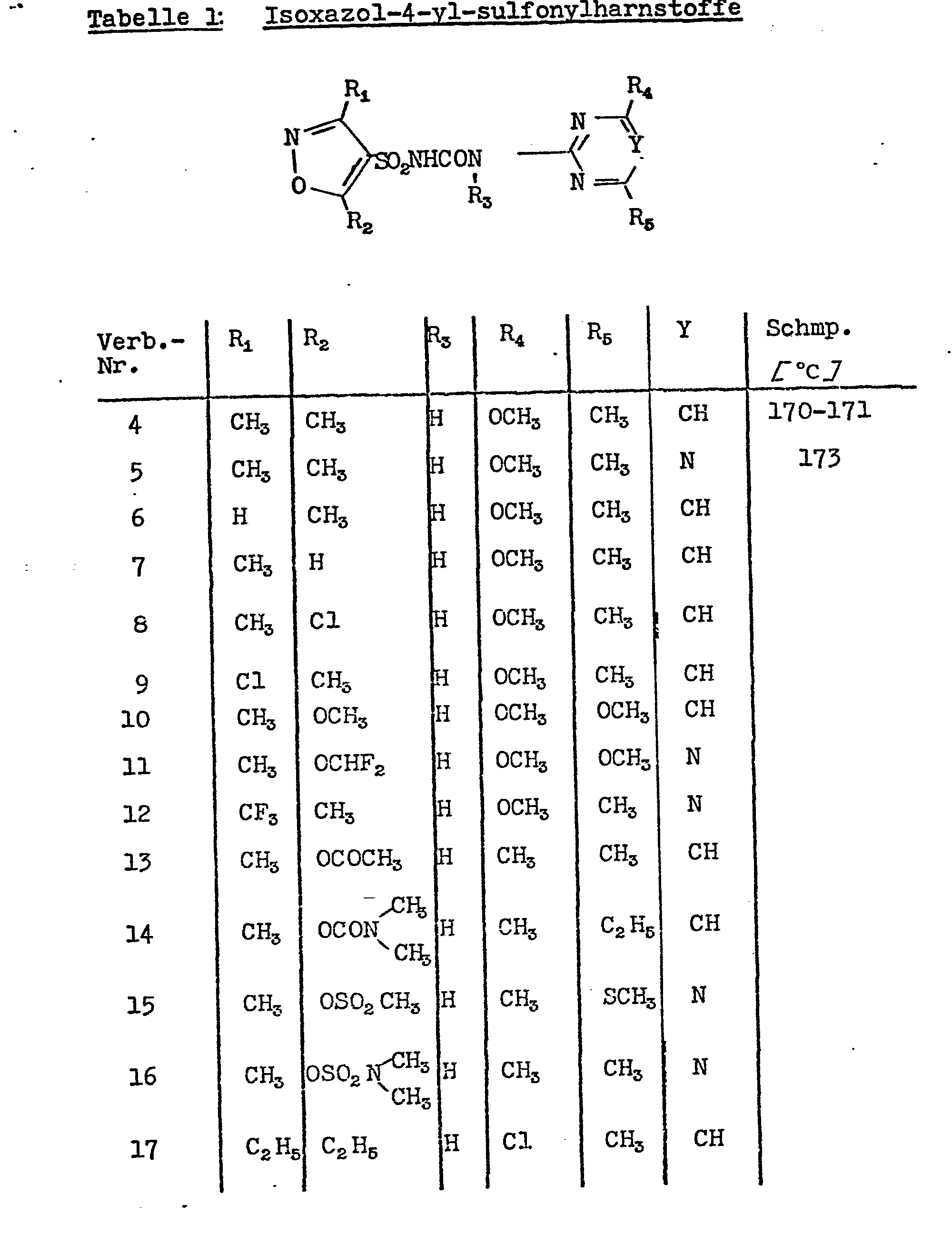

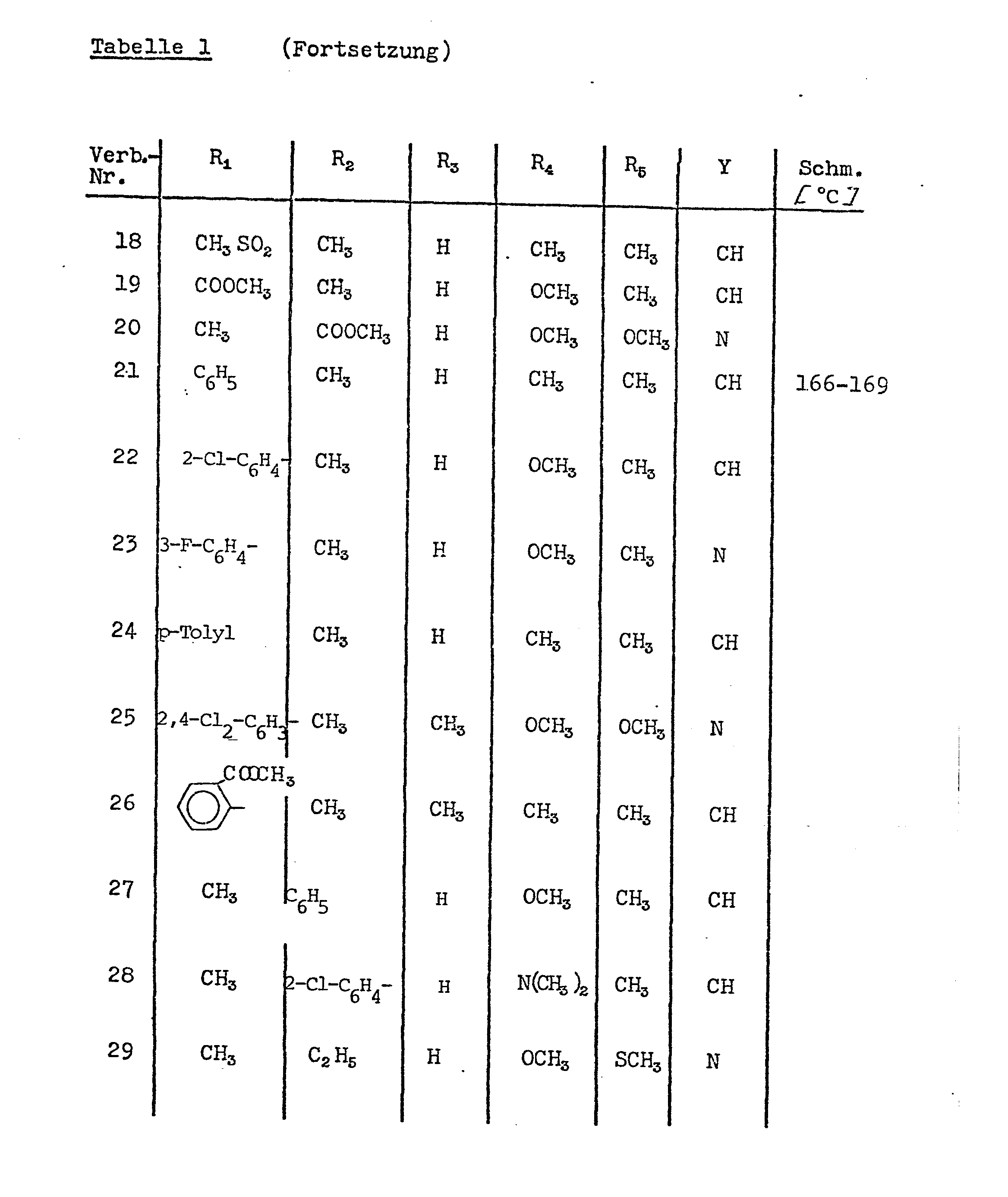

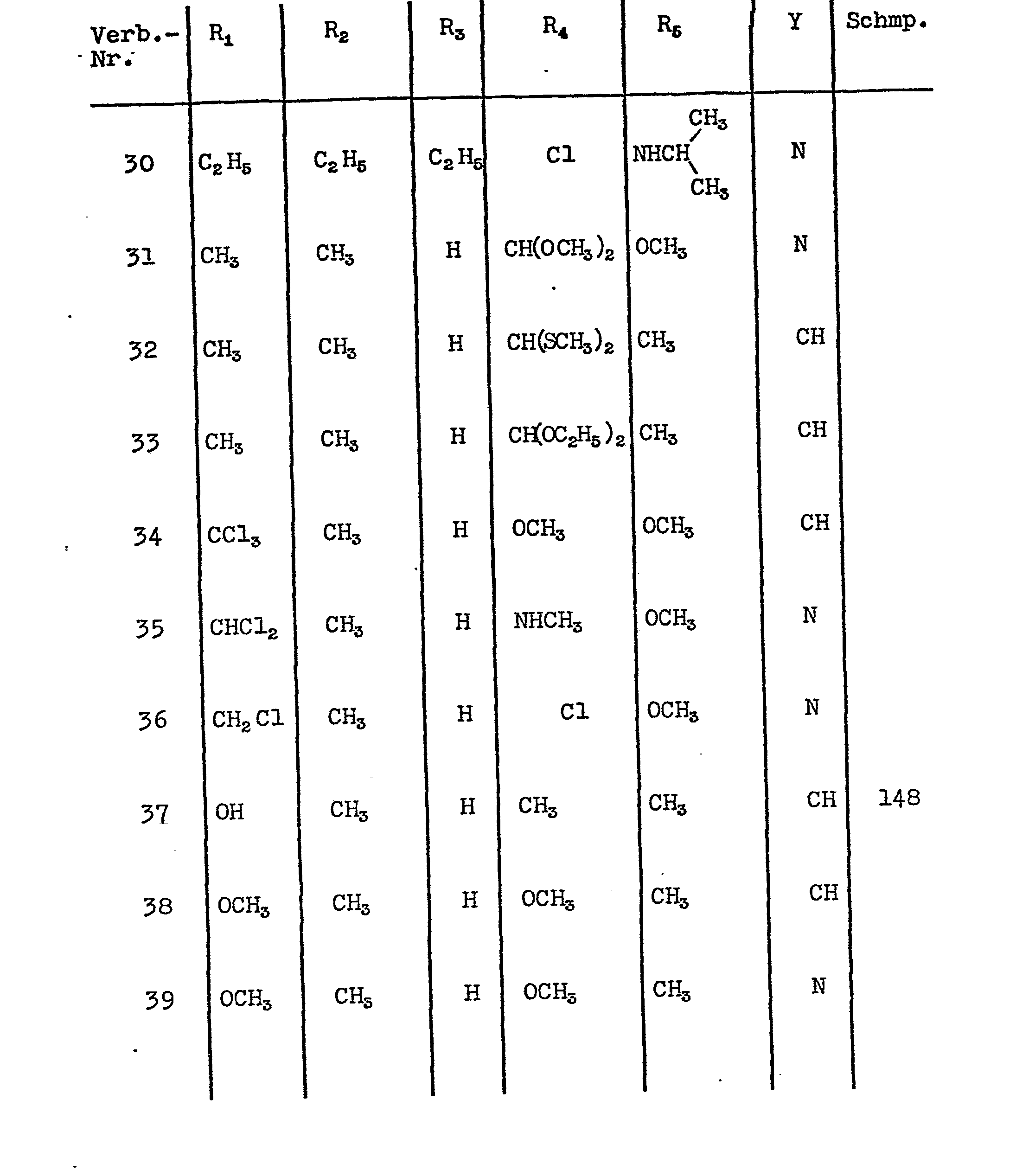

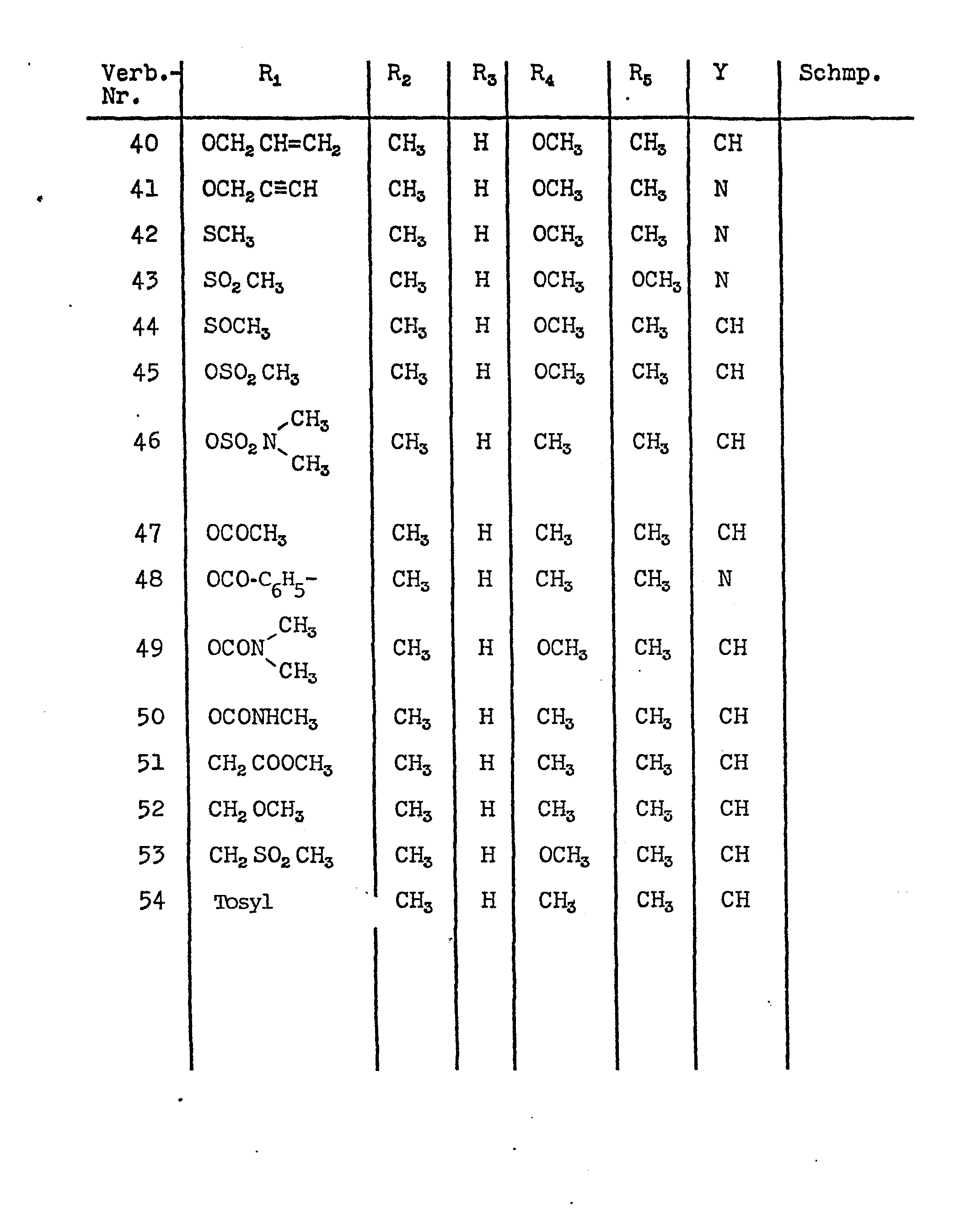

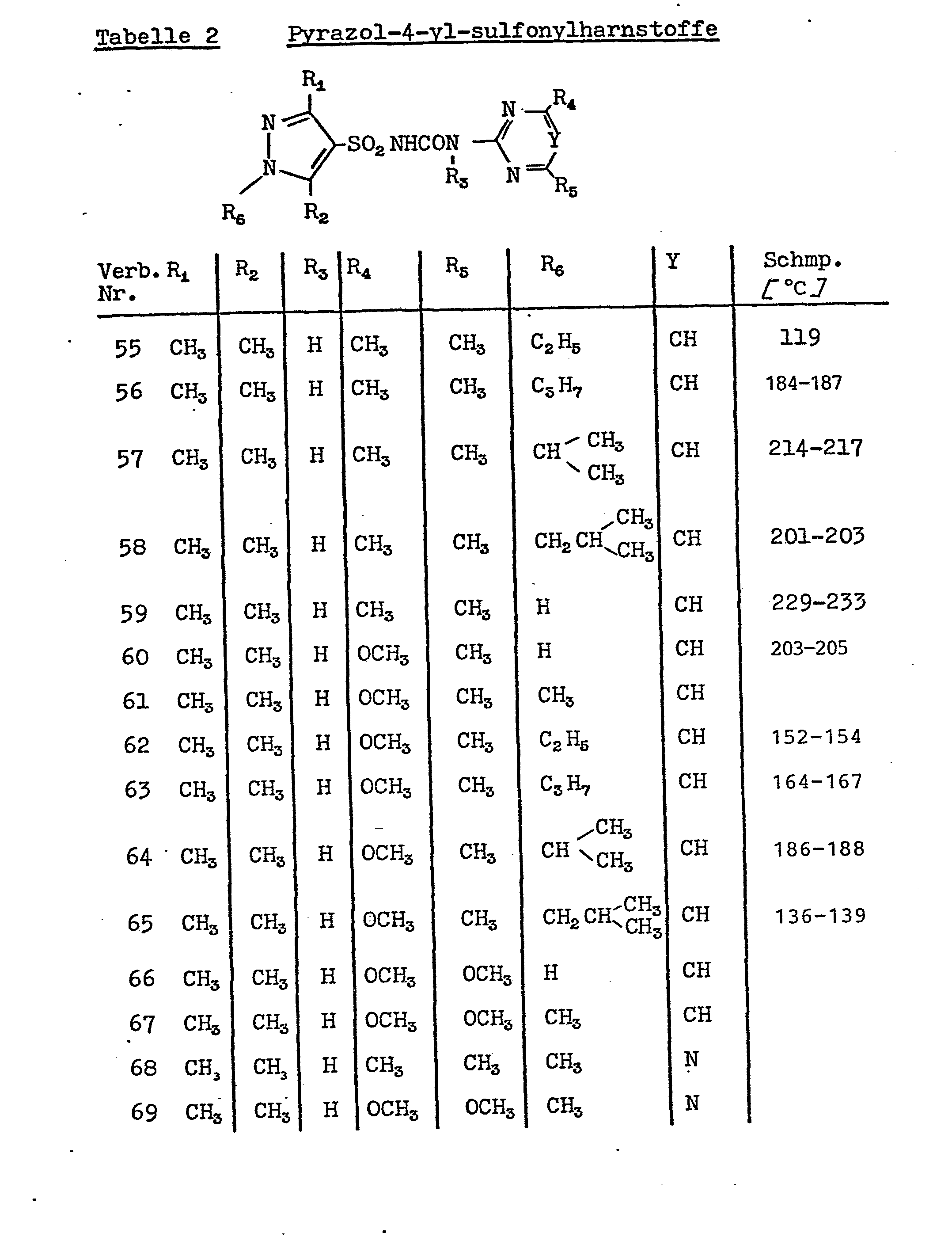

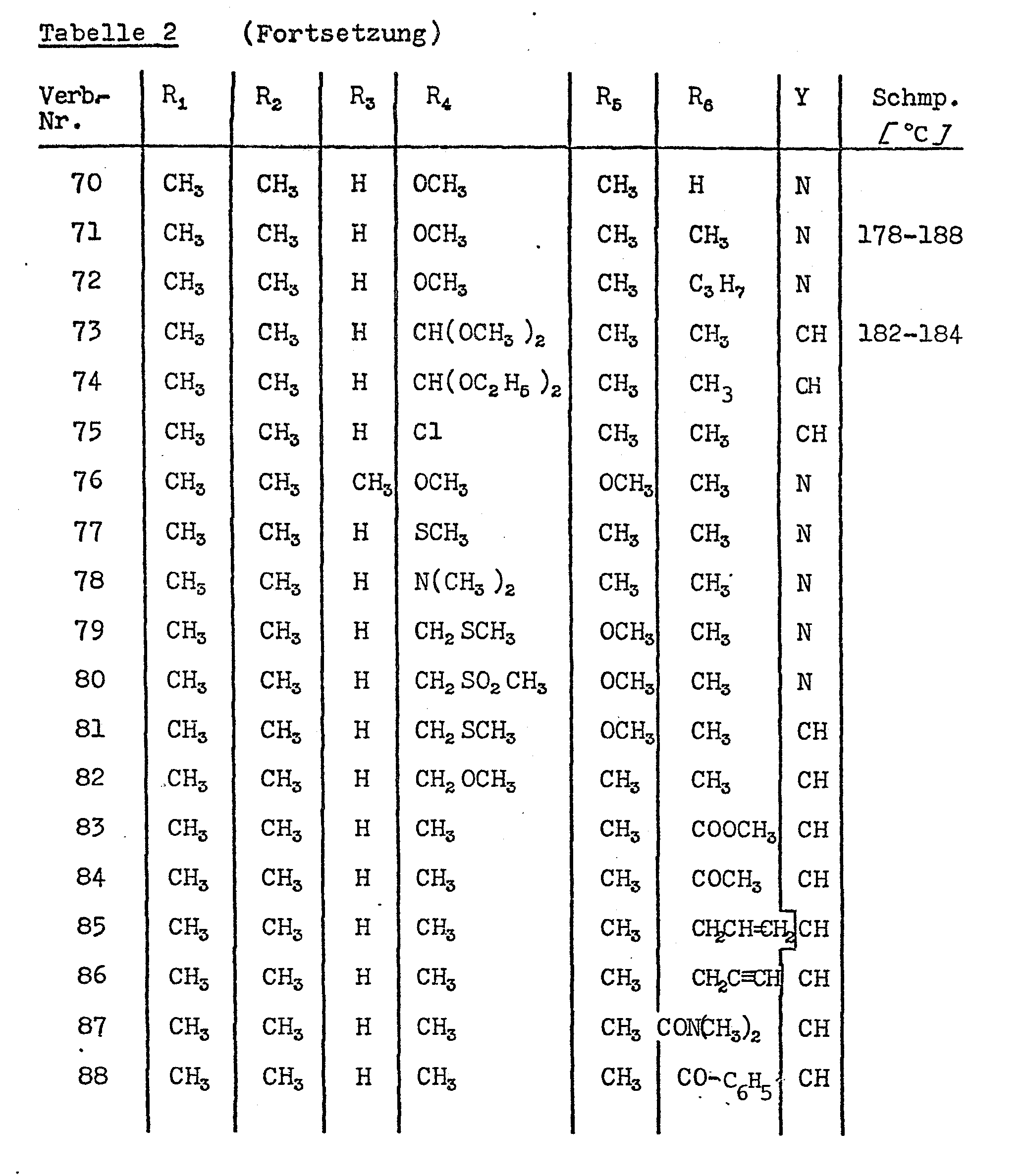

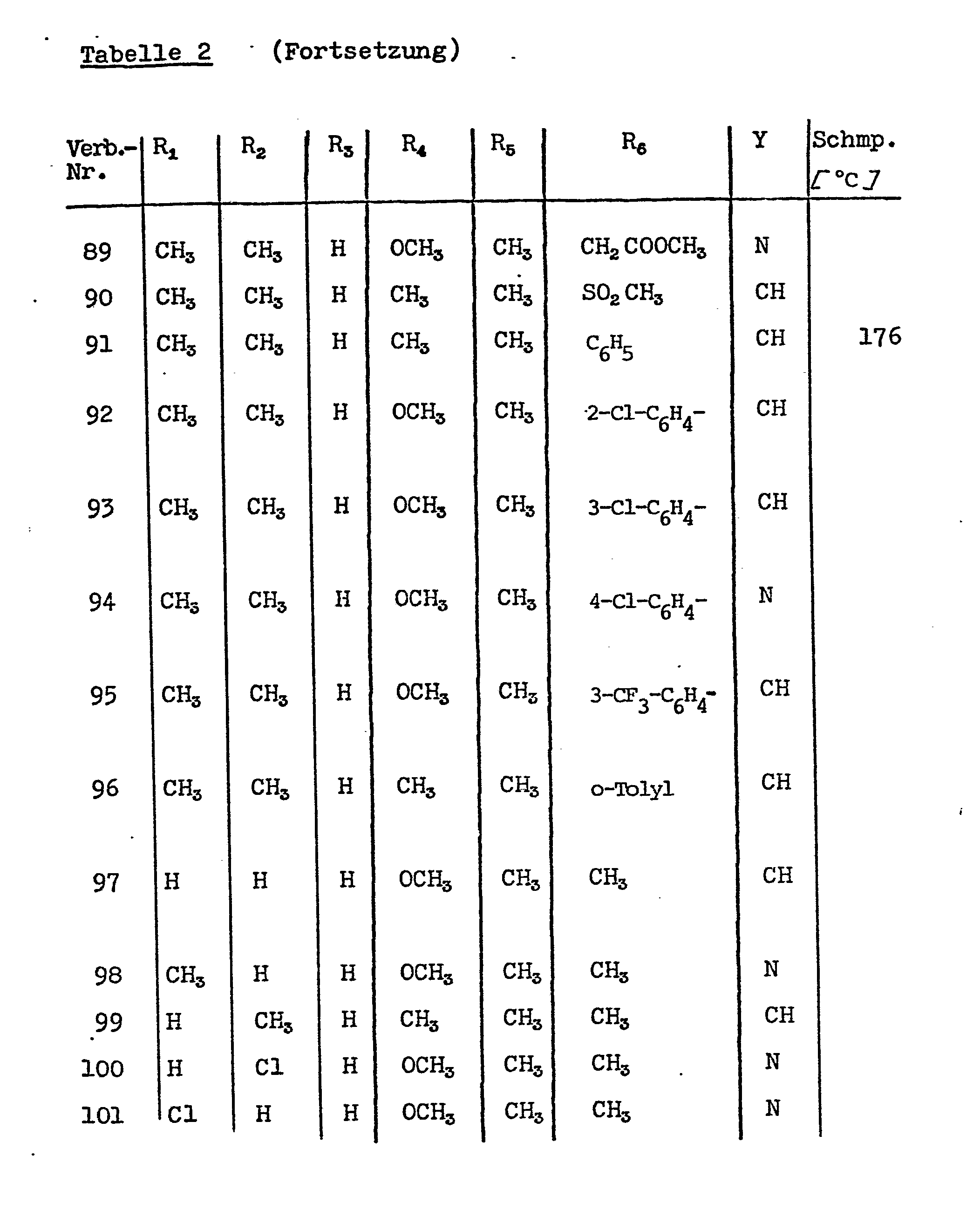

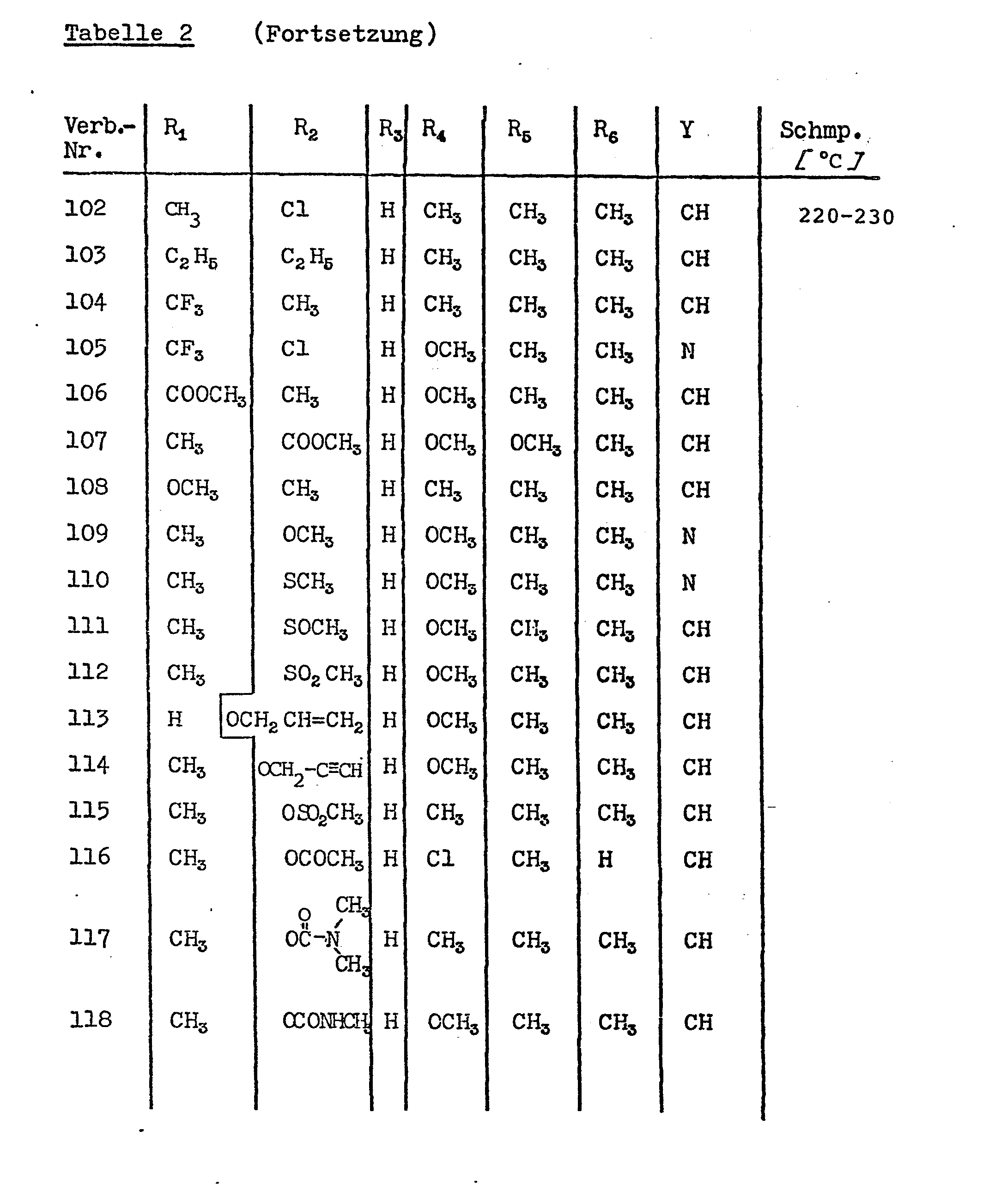

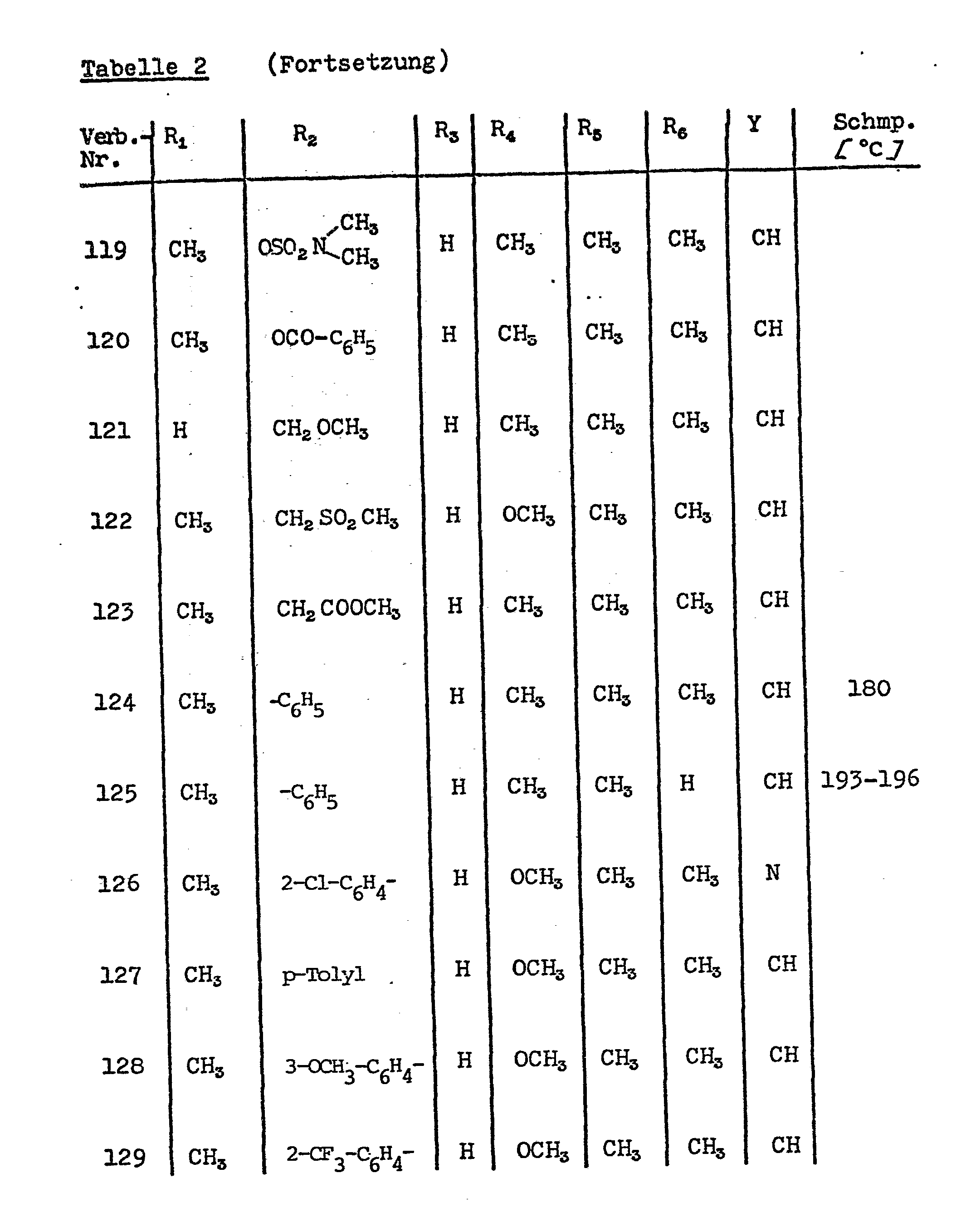

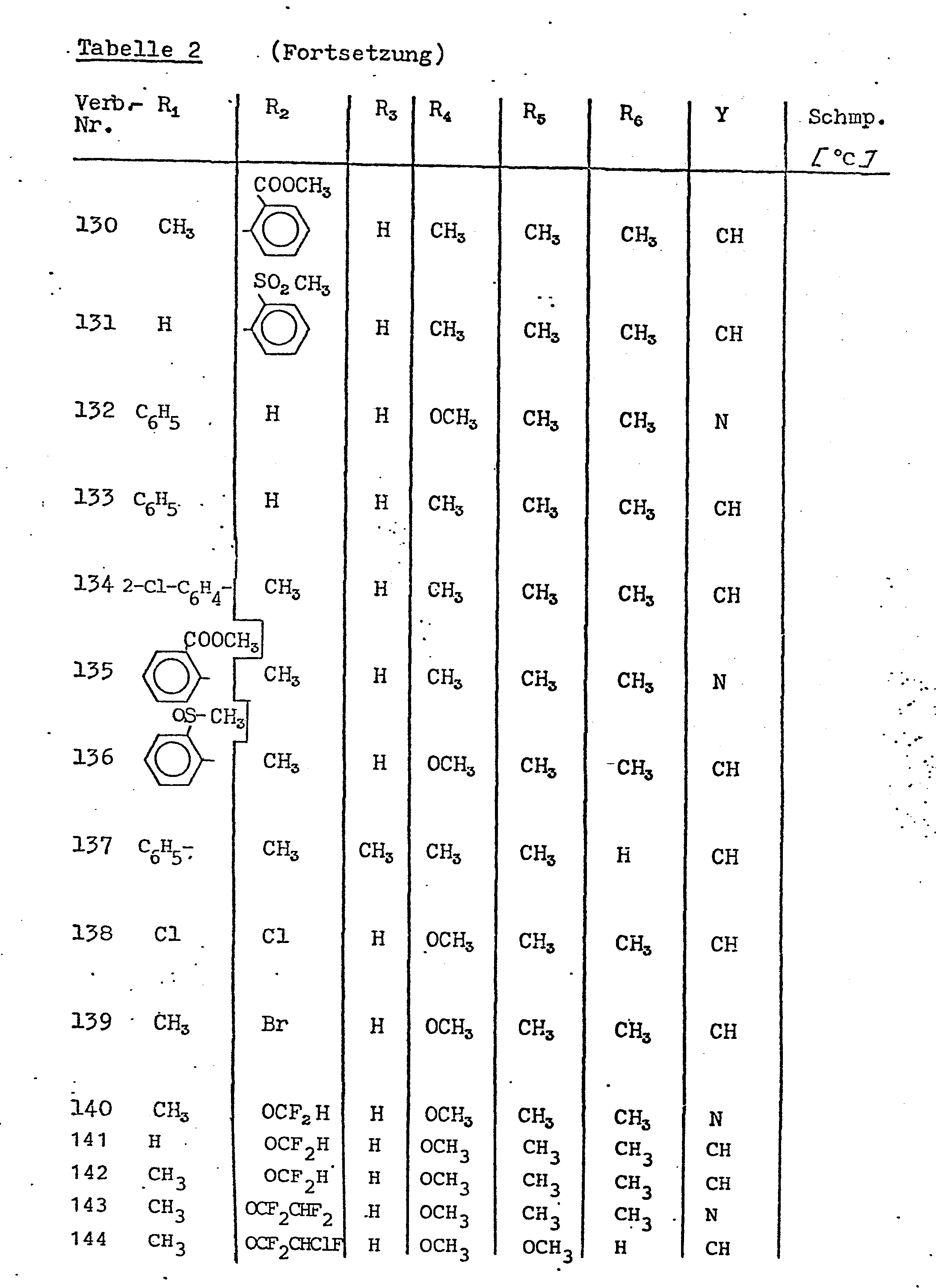

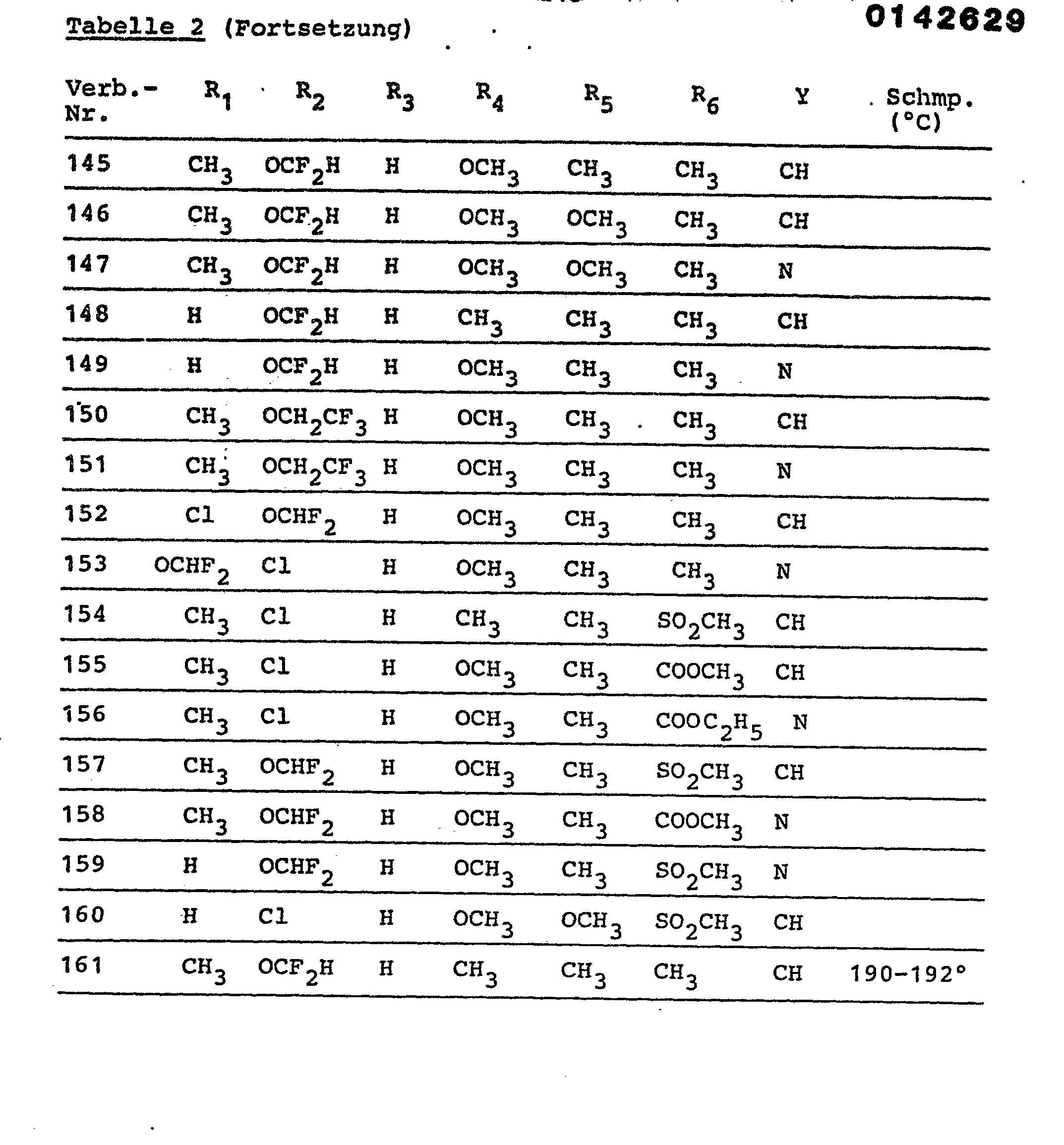

I. Herbizide Wirkung

Die vorliegenden erfindungsgemäßen Verbindungen weisen eine ausgezeichnete herbizide Wirksamkeit gegen ein breites Spektrum wirtschaftlich wichtiger mono- und dikotyler Schadpflanzen auf. Auch schwer bekämpfbare perennierende Wurzelunkräuter werden durch die Wirkstoffe gut erfaßt. Dabei ist es gleichgültig, ob die Substanzen in Vorsaat-, Vorauflauf- oder Nachauflaufspritzung ausgebracht werden. Werden die erfindungsgemäßen Verbindungen vor dem Keimen der Unkrautpflanzen im Vorsaat- oder Vorauflaufverfahren auf die Erdoberfläche appliziert, so wird das Auflaufen der Keimlinge nicht verhindert. Die Unkräuter wachsen bis zum Keimblattstadium heran, stellen jedoch dann ihr Wachstum ein und sterben schließlich nach 3 - 5 Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstopp ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräuter aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur Bekämpfung von unerwünschtem Pflanzenwuchs in landwirtschaftlichen Nutzpflanzungen.

Die Schädigung der Unkrautpflanzen bzw. die Kulturpflanzenverträglichkeit wurde in einem Schlüssel von 0-5 bonitiert.

Dabei bedeutet
0 = ohne Wirkung (Schaden)
1 = 0 - 20 % Wirkung
2 =20 - 40 % Wirkung
3 = 40 - 60 % Wirkung
4 = 60 - 80 % Wirkung
5 = 80 - 100 % Wirkung

### 1. Vorauflaufverfahren

Samen bzw. Rhizomstücke mono- und dikotyler Unkräuter wurden in Lehmerde in Plastiktöpfen (0 9 cm) ausgelegt und mit Erde abgedeckt. Die als benetzbare Pulver bzw. als Emulsionskonzentrate formulierten erfindungsgemäßen Verbindungen wurden in Form wässriger Suspensionen oder Emulsionen auf die Erdoberfläche appliziert. Die Wasseraufwandmenge pro Topf entsprach dabei umgerechnet 600 - 800 1/ha. Nach der Behandlung wurden die Versuchstöpfe im Gewächshaus aufgestellt und die Versuchspflanzen unter guten Wachstumsbedingungen (Temperatur: 23 ± 1 °C; rel. Luffeuchte 60 - 80 %) kultiviert. Nach ca. 3 Wochen wurde die Pflanzenschädigung optisch bonitiert. Als Vergleich dienten dabei unbehandelte Kontrollen. Wie aus den Werten der Tabelle 3 hervorgeht, weisen die erfindungsgemäßen Verbindungen im Vorauflaufverfahren eine zum Teil ausgezeichnete herbizide Wirksamkeit gegen wirtschaftlich bedeutende mono- und dikotyle Schadpflanzen auf.

### 2. Nachauflaufverfahren

Samen von mono- und dikotylen Unkräutern wurden in Töpfen ausgesät und im Gewächshaus unter guten Wachstumsbedingungen angezogen. 3 Wochen nach der Aussaat wurden die Versuchspflanzen im Dreiblattstadium behandelt. Die als Spritzpulver bzw. als Emulsionskonzentrate formulierten erfindungsgemäßen Präparate wurden in verschiedenen Dosierungen auf die grünen Pflanzenteile gesprüht und nach ca. 3 Wochen Standzeit im Gewächshaus unter optimalen Wachstumsbedingungen (Temperatur: 23 ± 1 °C ; rel. Luftfeuchte 60 - 80 %) die Wirkung der Präparate optisch im Vergleich zu unbehandelten Kontrollen bonitiert. Die erfindungsgemäßen Mittel wiesen eine gute herbizide Wirksamkeit gegen ein breites Spektrum wirtschaftlich wichtiger Unkräuter und Ungräser auf (Tabelle 4).

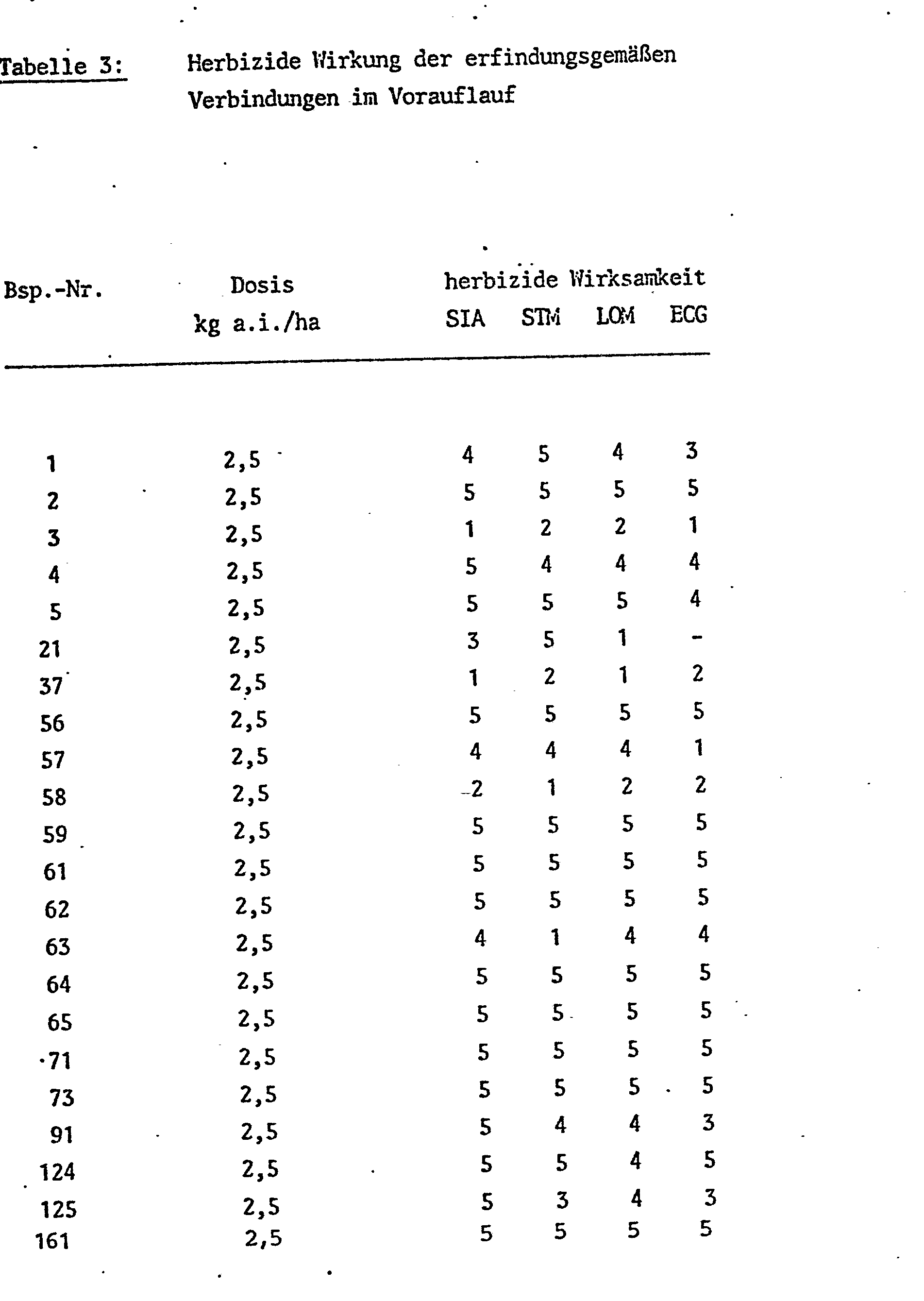

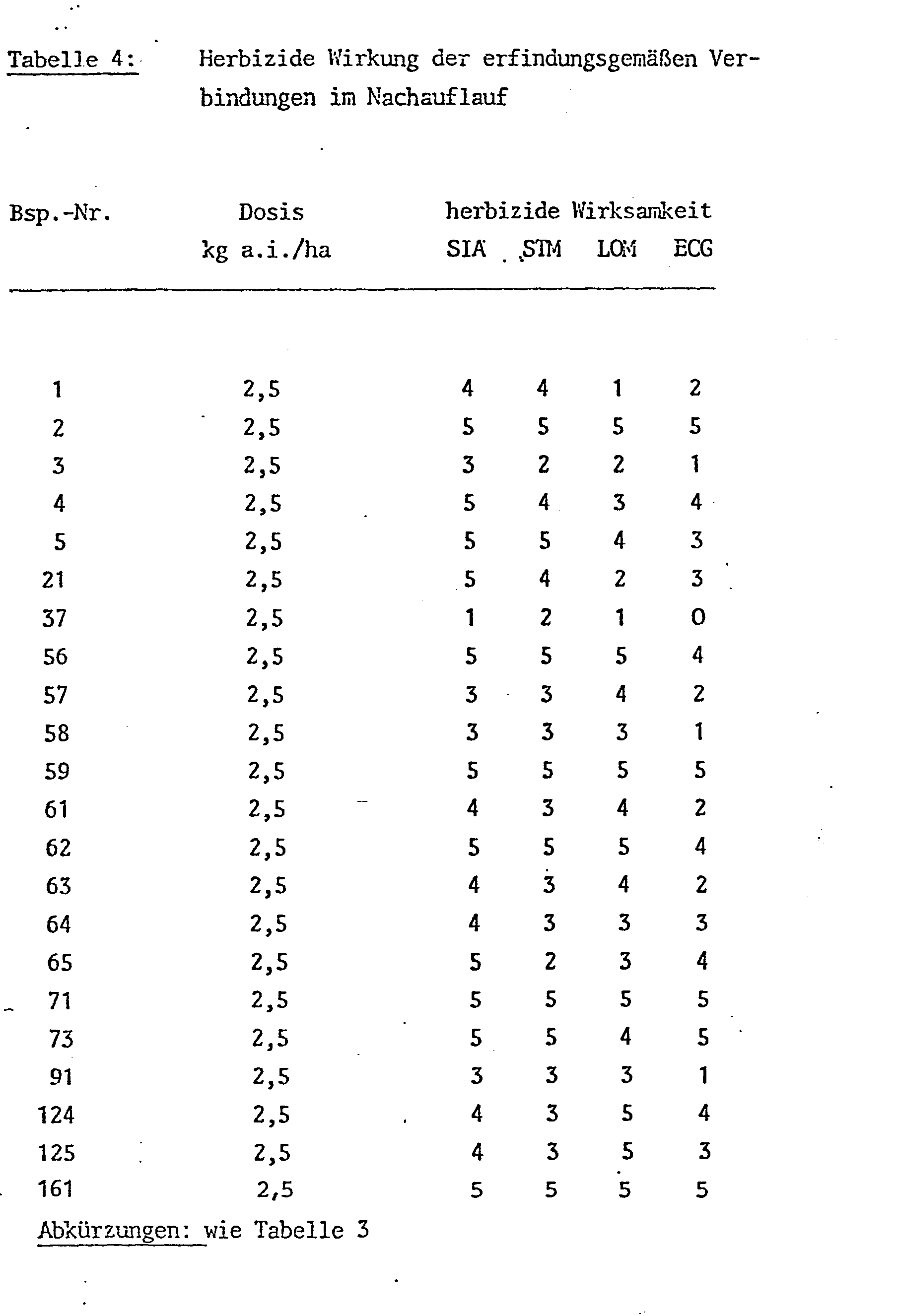

### II. Wachstumsregulatorische Wirksamkeit

### 1. Anregung der Äthylenfreisetzung

Calamondinorangen wurden 2 Minuten lang in Wirkstofflösungen von 2000 ppm Wirkstoffgehalt getaucht. Das von den Früchten gebildete Äthylen wurde danach täglich 5 Tage lang gaschromatisch erfaßt.

Die Ergebnisse der Tabelle 5 stellen einen Mittelwert aus insgesamt 3 Versuchsreihen dar.

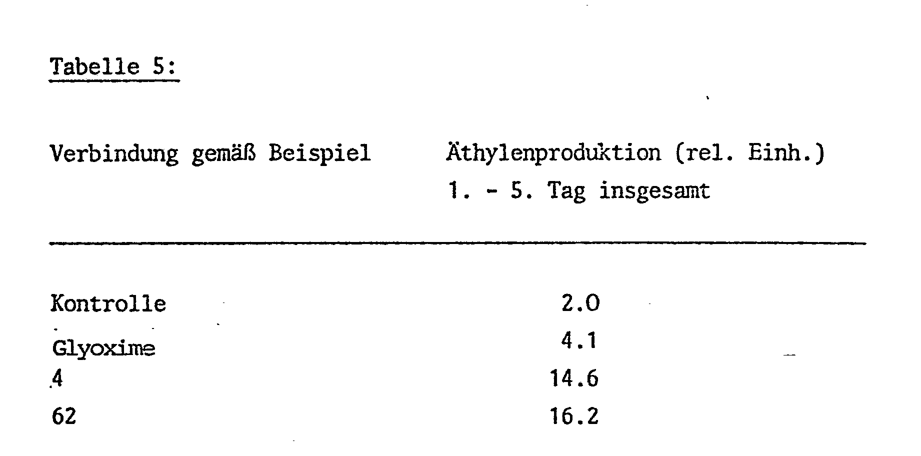

Die anspruchsgemäßen Verbindungen wiesen, wie in Tabelle 1 ersichtlich, eine wesentlich höhere Initial- und Dauerwirkung bei der Äthylenfreisetzung als das Vergleichsmittel .Glyoxime auf. Auch die insgesamt gebildete Äthylenmenge übersteigt deutlich die des Vergleichsmittels. Da Äthylen, das auch von der Pflanze gebildet wird, maßgeblich am Reifungs- und Abszissionsprozeß beteiligt ist, dient der Versuch als Beweis für die durch die anmeldungsgemäßen Verbindungen hervorgerufene beschleunigte und umfassende Ausbildung von Trennungsgewebe und damit für die Einleitung des Abszissionsvorgangs.

### 2. Abszissionswirkung bei Citruspflanzer.

Astpartien von Orangenbäumen mit mindestens 20 Früchten wurden kurz vor der Ernte mit Wirkstofflösungen bespritzt. Nach 7 Tagen erfolgte die Auswertung durch Messung der Haltekraft.

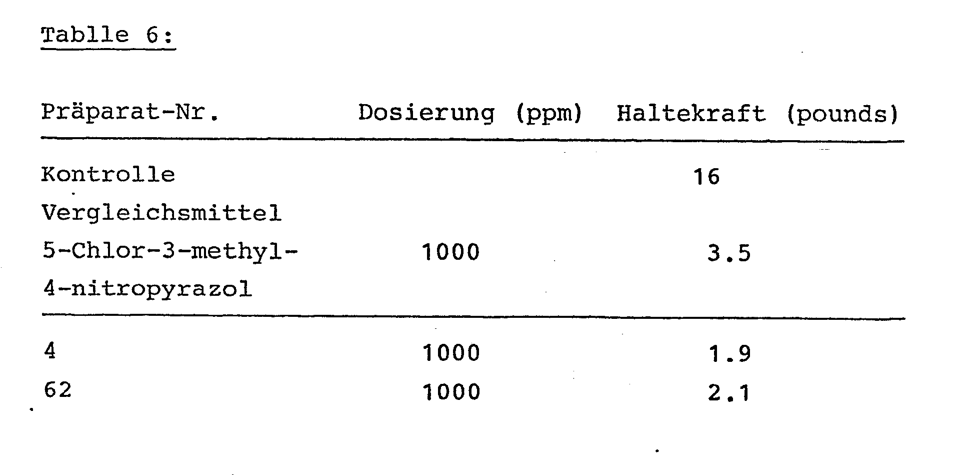

## Patentansprüche

1. Verbindungen der Formel I 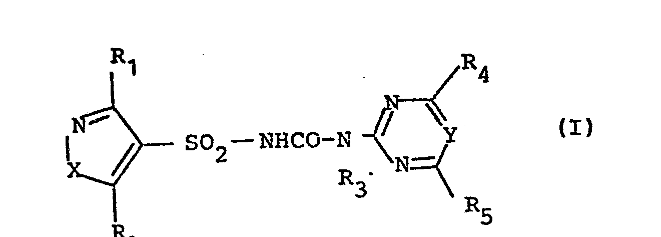 worin 2
X Sauerstoff oder NR₆,
Y CH oder Stickstoff,
R₁ und R₂ unabhängig voneinander Wasserstoff, Halogen, (C₁-C₆)Alkyl, (C₂-C₆)Alkenyl, (C₂-C₆)Alkinyl, die gegebenenfalls durch ein bis drei Halogenatome, (C₁-C₄)Alkoxy, (C₁-C₄)Alkylsulfenyl, (C₁-C₄) Alkylsulfinyl, (C₁-C₄)Alkylsulfonyl oder (C₁-C₄) Alkoxycarbonyl substituiert sind, OH, (C₁-C₄) Alkoxy, (C₂-C₄)Alkenyloxy, (C₂-C₄)Alkinyloxy, -OS0₂ (C₁-C₄)Alkyl, -OCO(C₁-C₄)Alkyl, -OCON [(C₁-C₄)Alkyl]₂, -OCONH(C₁-C₄)Alkyl, -OS0₂-C₆H₄-(4)-CH₃, -OS0₂N(C^{H}₃)₂, -OCO-C₆H₅, (C₁-C₄)Alkylsulfenyl,- (C₁-C₄)Alkylsulfinyl, (C₁-C₄)Alkylsulfonyl, -SO₂NH((C₁-C₄)Alkyl, -SO₂N[C₁-C₄)Alkyl]₂, einen Phenylrest, der gegebenenfalls durch Halogen, CF₃, (C₁-C₄)Alkyl, (C₁-C₄)Alkoxy, (C₁-C₄) Alkoxycarbonyl, (C₁-C₄)-Alkylsulfenyl, (C₁-C₄) Alkylsulfinyl oder (C₁-C₄)-Alkylsulfonyl substituiert ist, Benzyl, (C₁-C₄)Alkoxycarbonyl, (C₂-C₄) Alkenyloxycarbonyl, (C₂-C₄)Alkinyloxycarbonyl, Aminocarbonyl, (C₁-C₄)Alkylaminocarbonyl oder Di (C₁-C₄-alkyl)aminocarbonyl,
R₃ Wasserstoff oder (C₁-C₄)Alkyl,
R_{4'} R₅ unabhängig voneinander Wasserstoff, eine (C₁-C₄) Alkyl oder (C₁-C₄)Alkoxyrest, die gegebenenfalls ein bis dreifach durch Halogen oder 1 bis zweifach durch (C₁-C₂)Alkoxy oder (C₁-C₂)Alkylthio substituiert sein können, Halogen, (C₁-C₄)Alkylthio, (C₁-C₄) Alkylamino oder (C₁-C₄)Dialkylamino,
und für X = NR₆
R₆ Wasserstoff, (C₁-C₆)Alkyl, (C₂-C₄)Alkenyl, (C₂-C₄)Alkinyl, einen Phenylrest, der gegebenenfalls durch Halogen, (C₁-C₄)Alkyl, CF₃, (C₁-C₄) Alkoxycarbonyl oder N0₂ substituiert ist, -CO(C₁-C₄) Alkyl, -COO(C₁-C₆)-Alkyl, -CONH(C₁-C₆)Alkyl, -CON[(C₁-C₄)Alkyl]₂, -CO-C₆H₅, -CH₂-COO(C₁-C₄) Alkyl, -SO₂(C₁-C₄)Alkyl oder -S0₂-C₆H₄-(⁴)^{CH}₃ bedeuten sowie deren nicht^{p}hytotoxische Salze.

2. Verfahren zur Herstellung der Verbindungen der Formel I, dadurch gekennzeichnet, daß man
a) Verbindungen der Formel (II) 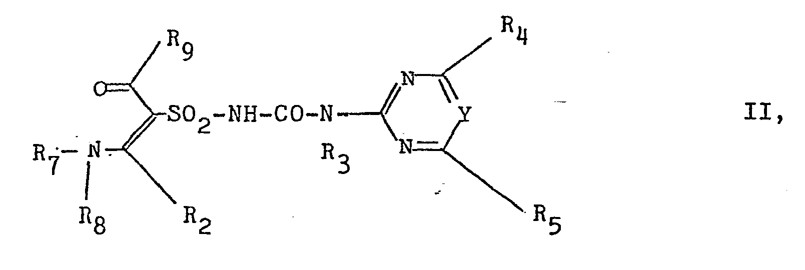 worin
R₇ und R₈ unabhängig voneinander Wasserstoff, (C₁-C₈) (C₃-C₇)-Cycloalkyl, Phenyl, welches gegebenenfalls ein- oder mehrfach durch Halogen, (C₁-C₄) Alkyl, (C₁-C₄)-Alkoxy, (C₁-C₄)Alkylthio, (C₁-C₄)Alkoxycarbonyl, CF₃, N0₂ oder CH₃SO₂-substituiert sein kann, oder die Reste R₇ und R₈ zusammen mit dem gemeinsamen Stickstoffatom einen 5- bis 7-gliedrigen aliphatisehen Ring bilden, worin eine CH₂-Gruppe gegebenenfalls durch Sauerstoff, NH oder N(C₁-C₄)Alkyl ersetzt sein kann,
R₉ Wasserstoff, (C₁-C₈)Alkyl, (C₃-C₇)-C^{y}clo- alkyl oder Phenyl, welches gegebenenfalls ein-oder mehrfach durch Halogen, (C₁-C₄)Alkyl, (C₁-C₄)Alkoxy, (C₁-C₄)Alkylthio, CF₃, N0₂ oder (C₁-C₄)Alkoxycarbonyl substituiert sein kann und
R₂, _{R3}, R₄, R₅ und X die Bedeutungen wie in Formel (I) besitzen, mit Verbindungen der Formel (III) 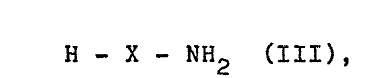 worin X die Bedeutung wie in Formel I besitzt, umsetzt oder
b) Verbindungen der Formel I mit X = NR₆ und R₆ = H durch Alkylierung oder Acylierung der NH-Gruppe des Pyrazolringes in andere Verbindungen der Formal I überführt, oder
c) Verbindungen der Formel I mit R₂ = OH durch Alkylierung, Acylierung oder Halogenierung der heterocyclischen OH-Gruppierung in andere Verbindungen der Formel (I) überführt.

3. Herbizide und wachstumsregulierende Mittel, gekennzeichnet durch einen Gehalt an Verbindungen der Formel I gemäß Anspruch 1.

4. Verwendung von Verbindungen der Formel I von Anspruch 1 zur Bekämpfung von unerwünschtem Pflanzenwuchs und zur Wachstumsregulierung.

5. Verfahren zur Bekämpfung von unerwünschtem Pflanzenwuchs bzw. zur Wachstumsregulierung, dadurch gekennzeichnet, daß man auf die zu behandelnden Pflanzen oder die Anbaufläche eine wirksame Menge einer Verbindung der Formel I von Anspruch 1 aufbringt.

Patentansprüche Österreich 1. Verfahren zur Herstellung von Verbindungen der Formel I 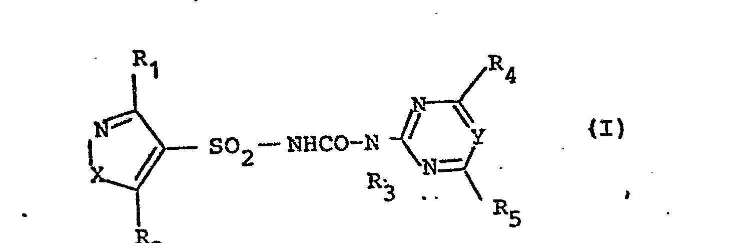 worin
X Sauerstoff oder NR₆,
Y CH oder Stickstoff,
R₁ und R₂ unabhängig voneinander Wasserstoff, Halogen, (C₁-C₆)Alkyl, (C₂-C₆)Alkenyl, (C₂-C₆)Alkinyl, die gegebenenfalls durch ein bis drei Halogenatome, (C₁-C₄)Alkoxy, (C₁-C₄)Alkylsulfenyl, (C₁-C₄) Alkylsulfinyl, (C₁-C₄)Alkylsulfonyl oder (C₁-C₄) Alkoxycarbonyl substituiert sind, OH, gegebenenfalls halogeniertes (C₁-C₄)Alkoxy, (C₂-C₄)Alkenyloxy , (C₂-C₄)Alkinyloxy, -OSO₂ (C₁-C₄)Alkyl, -OCO(C₁-C₄)Alkyl, -OCON [(C₁-C₄)Alkyl]₂, -OCONH(C₁-C₄)Alkyl, -OSO₂-C₆H₄-(4)-CH_{3'} -OS02N(CH3)2, -OCO-C₆H₅, (C₁-C₄)Alkylsulfenyl, (C₁-C₄)Alkylsulfinyl, (C₁-C₄)Alkylsulfonyl, -SO₂NH((C₁-C₄)Alkyl, -SO₂N [(C₁-C₄)Alkyl]₂, einen Phenylrest, der gegebenenfalls durch Halogen, CF₃, (C₁-C₄)Alkyl, (C₁-C₄)Alkoxy, (C₁-C₄) Alkoxycarbonyl, (C₁-C₄)-Alkylsulfenyl, (C₁-C₄) Alkylsulfinyl oder (C₁-C₄)-Alkylsulfonyl substituiert ist, Benzyl, (C₁-C₄)Alkoxycarbonyl, (C₂-C₄) Alkenyloxycarbonyl, (C₂-C₄)Alkinyloxycarbonyl, Aminocarbonyl, (C₁-C₄)Alkylaminocarbonyl oder Di (C₁-C₄-alkyl)aminocarbonyl,
R₃ Wasserstoff oder (C₁-C₄)Alkyl,
R₄, R₅ unabhängig voneinander Wasserstoff, eine (C₁-C₄) Alkyl oder (C₁-C₄)Alkoxyrest, die gegebenenfalls ein bis dreifach durch Halogen oder 1 bis zweifach durch (C₁-C₂)Alkoxy oder (C₁-C₂)Alkylthio substituiert sein können, Halogen, (C₁-C₄)Alkylthio, (C₁-C₄) Alkylamino oder (C₁-C₄)Dialkylamino,
und für X = NR6
R₆ Wasserstoff, (C₁-C₆)Alkyl, (C₂-C₄)Alkenyl, (C₂-C₄)Alkinyl, einen Phenylrest, der gegebenenfalls durch Halogen, (C₁-C₄)Alkyl, CF₃, (C₁-C₄) Alkoxycarbonyl oder N0₂ substituiert ist, -CO(C₁-C₄) Alkyl, -COO(C₁-C₆)-Alkyl, -CONH(C₁-C₆)Alkyl, -CON[(C₁-C₄)Alkyl]₂, -CO-C₆H₅, -CH₂-COO(C₁-C₄) Alkyl, -SO₂(C₁-C₄)Alkyl oder -SO₂-C₆H₄-(4)CH₃ bedeuten sowie deren nicht^{p}hytotoxische Salze,
dadurch gekennzeichnet, daß man
a) Verbindungen der Formel (II) 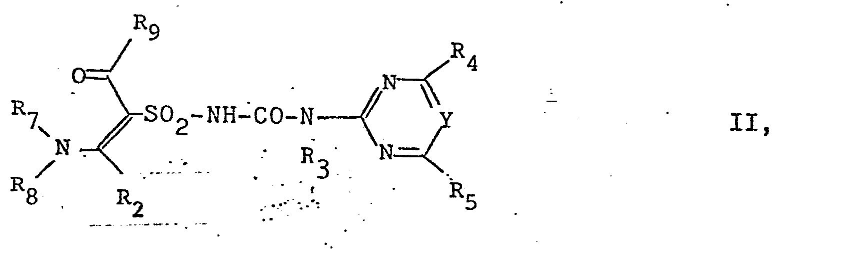 worin
R₇ und R₈ unabhängig voneinander Wasserstoff, (C₁-C₈) (C₃-C₇)-Cycloalkyl, Phenyl, welches gegebenenfalls ein- oder mehrfach durch Halogen, (C₁-C₄) Alkyl, (C₁-C₄)-Alkoxy, (C₁-C₄)Alkylthio, (C₁-C₄)Alkoxycarbonyl, CF₃, NO₂ oder CH₃SO₂-substituiert sein kann, oder die Reste R₇ und R₈ zusammen mit dem gemeinsamen Stickstoffatom einen 5- bis 7-gliedrigen aliphatisehen Ring bilden, worin eine CH₂-Gruppe gegebenenfalls durch Sauerstoff, NH oder N(C₁-C₄)Alkyl ersetzt sein kann,
R₉ Wasserstoff, (C₁-C₈)Alkyl, (C₃-C₇)-Cycloalkyl oder Phenyl, welches gegebenenfalls ein-oder mehrfach durch Halogen, (C₁-C₄)Alkyl, (C₁-C₄)Alkoxy, (C₁-C₄)Alkylthio, CF₃, NO₂ oder (C₁-C₄)Alkoxycarbonyl substituiert sein kann und
R₂, R₃, R₄, R₅ und X die Bedeutungen wie in Formel (I) besitzen,
mit Verbindungen der Formel (III) 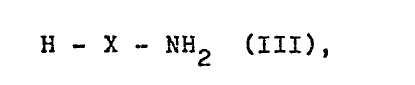 worin X die Bedeutung wie in Formel I besitzt, umsetzt und gegebenenfalls Verbindung der Formel I mit X= N oder R₂= OH durch Derivatisierung in andere Verbindungen der Formel I überführt.

2. Herbizide und wachstumsregulierende Mittel, gekennzeichnet durch einen Gehalt an einer Verbindung der Formel gemäß Anspruch 1.

3. Verwendung von Verbindungen der Formel I von Anspruch 1 zur Bekämpfung von unerwünschtem Pflanzenwuchs und zur Wachstumsregulierung.

4. Verfahren zur Bekämpfung von unerwünschtem Pflanzenwuchs bzw. zur Wachstumsregulierung, dadurch gekennzeichnet, daß man auf die zu behandelnden Pflanzen oder die Anbaufläche eine wirksame Menge einer Verbindung der Formel I von Anspruch 1 aufbringt.
